# FASCICULE DE BREVET EUROPEEN

(11) **EP 2 066 409 B1**
(45) Date de publication et mention de la délivrance du brevet: **31.03.2010**
(21) Numéro de dépôt: 07823756.7
(22) Date de dépôt: 31.08.2007
(51) Int. Cl.: A61Q 19/06, A61K 8/97, A61P 3/06, A61K 36/00

(54) **UTILISATION D'UN EXTRAIT DE QUINOA COMME ACTIF COSMETIQUE ET PHARMACEUTIQUE AMINCISSANT ET/OU COMME ACTIF PREVENANT DE LA FORMATION DE NOUVELLES GRAISSES DANS LE CORPS HUMAIN**
VERWENDUNG EINES QUINOA-EXTRAKTS ALS KOSMETISCHES UND PHARMAZEUTISCHES SCHLANKHEITSMITTEL UND/ODER ALS MITTEL ZUR VERHINDERUNG DER FORMIERUNG NEUER FETTE IM MENSCHLICHEN KÖRPER
USE OF QUINOA EXTRACT AS COSMETIC AND PHARMACEUTIC SLIMMING AGENT AND/OR AS AN AGENT PREVENTING THE FORMATION OF NEW FATS IN THE HUMAN BODY

(30) Priorité: 18.09.2006 FR 0653790; 18.09.2006 FR 0653801
(43) Date de publication de la demande: 10.06.2009
(73) Titulaire: Societe D'Exploitation De Produits Pour Les Industries Chimiques Seppic, 75007 Paris (FR)
(72) Inventeur: GARCIA, Christine, 81100 Castres (FR); STOLTZ, Corinne, Thiais 94320 (FR)
(74) Mandataire: Conan, Philippe Claude
(86) Numéro de dépôt international: PCT/FR2007/051859
(87) Numéro de publication internationale: WO 2008/034989

(56) Documents cités:
- WO-A-96/03998
- FR-A- 2 844 449
- GB-A- 2 391 476
- US-A1- 2005 271 751

## Description

La présente invention a pour objet une nouvelle utilisation d'actifs cosmétiques pour amincir le corps humain et/ou pour prévenir la formation de graisse dans le corps humain.

Une partie de la graisse du corps humain est stockée sous forme de triglycérides, dans des cellules du tissu gras du derme, appelés adipocytes. L'amincissement rend compte d'une diminution de la graisse stockée dans les adipocytes. Ce processus nécessite une étape prioritaire qui a lieu à l'intérieur de ces cellules et qui consiste en l'hydrolyse des triglycérides en acides gras et glycérol. Ce phénomène s'appelle la lipolyse. La plupart des formulations cosmétiques amincissantes commercialisées aujourd'hui, contiennent au moins un composé possédant une activité lipolytique. Le plus fréquemment utilisé est la caféine mais la théophylline est aussi connue pour posséder une telle propriété.

Au contraire, lors de la prise de poids, les adipocytes peuvent se remplir de graisse dans le cadre d'un processus dit d'atrophie adipocytaire mais il peut également se produire un phénomène de recrutement de nouveaux adipocytes par différenciation de pré-adipocytes en adipocytes matures, capables de stocker les triglycérides selon le processus d'hyperplasie adipocytaire. Un actif permettant de prévenir la prise de poids peut donc être sélectionné sur sa capacité à empêcher le recrutement de nouveaux adipocytes. La demande de brevet français publiée sous le numéro FR 2 844 449 divulgue l'utilisation d'un extrait d'une algue brune du genre "*Halopteris*", dans une composition destinée à limiter l'expansion des tissus adipeux par son effet sur la différenciation pré-adipocytaire.

Le Tumoral Necrosis Factor ou TNFα, est largement décrit dans la littérature scientifique comme inhibiteur de cette maturation des adipocytes mais son utilisation n'est pas autorisée dans des formulations cosmétiques. Il n'existe donc pas de molécule active connue pour un usage en cosmétique dans l'état de la technique, constituant un inhibiteur de référence de la différenciation des pré-adipocytes.

A l'occasion de leur recherche de nouveaux actifs à activité lipolytique, et/ou permettant d'inhiber la différenciation des pré-adipocytes, afin de prévenir la formation de nouvelles graisses qui aient une bonne compatibilité avec la peau, les inventeurs ont mis en évidence que les extraits de Quinoa, habituellement commercialisés par les industries cosmétiques pour leurs propriétés hydratante et décongestionnante, avaient à la fois un effet lipolytique plus efficace que celui de la caféine et un effet inhibiteur de la différenciation des pré-adipocytes.

C'est pourquoi, selon un premier aspect, l'invention a pour objet l'utilisation d'un extrait de Quinoa, comme actif amincissant, et/ou comme actif permettant d'inhiber la formation de nouvelles graisses dans le corps humain, dans une composition contenant un milieu cosmétiquement acceptable.

Par Quinoa, on désigne dans la présente demande de brevet, la plante quinoa (*Chenopodium quinoa*) ou de ses variantes génétiquement modifiées. Les extraits de Quinoa mis en oeuvre dans l'invention objet de la présente demande de brevet, sont généralement disponibles dans le commerce sous forme d'extraits liquides comme les extraits aqueux, les extraits glycoliques, dans lesquels le solvant est par exemple le glycol, le propylène glycol ou le butylène glycol, les extraits hydro-glycoliques, les extraits alcooliques dans lesquels l'alcool est par exemple l'éthanol alcoolique ou les extraits hydroalcooliques. De tels extraits contiennent généralement entre 0,01 % et 10 % massique d'extrait sec.

L'invention a aussi pour objet un procédé de traitement non thérapeutique du corps humain destiné à l'amincir et/ou destiné à inhiber la formation de nouvelles graisses, **caractérisé en ce que** l'on y applique une composition contenant un milieu cosmétiquement acceptable et une quantité efficace d'extrait de Quinoa.

Par quantité efficace on désigne dans le cadre de la présente invention, un pourcentage massique en extrait sec par rapport à la composition finale, compris entre 0,0001 % massique et 2,0 % massique, préférentiellement entre 0,0001 % massique et 0,9 % massique.

L'invention a aussi pour objet l'utilisation d'un extrait de quinoa, tel que défini précédemment, pour préparer un médicament à activité lipolytique, destiné à induire l'amincissement du corps humain.

L'invention a aussi pour objet l'utilisation d'un extrait de quinoa, tel que défini précédemment, pour préparer un médicament à activité inhibitrice de différenciation des pré-adipocytes, destiné à prévenir la formation de nouvelles graisses du corps humain et/ou animal.

Dans les compositions définies ci-dessus, l'extrait de quinoa est généralement mis en oeuvre en une quantité suffisante pour qu'il y ait entre 0,0001 % et 2 % massique de composition en extrait sec, plus particulièrement entre 0,0005 % à 0,9 % massique de composition en extrait sec et tout particulièrement entre 0,001 % et 0,6 % massique de composition en extrait sec.

Comme le montre l'étude expérimentale suivante, les extraits de Quinoa mis en oeuvre dans les traitements cosmétique ou thérapeutique définis précédemment, se caractérisent de façon inattendue, par une activité lipolytique supérieure aux compositions de l'état de la technique et par une aptitude à prévenir la formation de graisse dans le corps humain. Ils sont donc de façon générale, appropriés aux traitements amincissants du corps humain.

Les compositions mises en oeuvre dans lesdits traitements, se présentent généralement sous forme de solutions aqueuses ou hydroalcooliques diluées, sous forme d'émulsions simples ou multiples, telles que les émulsions eau dans huile (E/H), huile dans eau (H/E) ou eau dans huile dans eau (E/H/E), dans lesquelles l'huile est de nature végétale ou minérale ou sous forme de poudre. Elles peuvent aussi être dispersées ou imprégnées sur du textile ou sur des matériaux non tissés qu'il s'agisse de lingettes, de serviettes en papier ou de vêtements.

Les compositions mises en oeuvre dans lesdits traitements, sont administrées au sujet sous les formes classiques utilisées en cosmétique et en pharmacie ; il s'agit plus particulièrement des administrations topique, orale ou parentérale.

De façon générale, les extraits de quinoa, sont associés à de nombreux types d'adjuvants ou de principes actifs utilisés dans les formulations cosmétiques, qu'il s'agisse, de corps gras, de solvants organiques, d'épaississants, de gélifiants, d'adoucissants, d'antioxydants, d'opacifiants, de stabilisants, de moussants, de parfums, d'émulsionnants, ioniques ou non, de charges, de séquestrants, de chélateurs, de conservateurs, de filtres chimiques ou de filtres minéraux, d'huiles essentielles, de matières colorantes, de pigments, d'actifs hydrophiles ou lipophiles, d'humectants, par exemple la glycérine, de conservateurs, de colorants, de parfums, d'actifs cosmétiques, de filtres solaires minéraux ou organiques, de charges minérales comme les oxydes de fer, oxydes de titane et le talc, de charges synthétiques comme les nylons et les poly(méthacrylate de méthyle) réticulés ou non, d'élastomères silicone, de séricites ou d'extraits de plantes ou encore de vésicules lipidiques ou tout autre ingrédient habituellement utilisé en cosmétique.

Comme exemples d'huiles que l'on peut associer aux extraits de quinoa, on peut citer, les huiles minérales telles que l'huile de paraffine, l'huile de vaseline, les isoparaffines ou les huiles blanches minérales, les huiles d'origine animale, telles que le squalène ou le squalane, les huiles végétales, telles que l'huile d'amandes douces, l'huile de coprah, l'huile de ricin, l'huile de jojoba, l'huile d'olive, l'huile de colza, l'huile d'arachide, l'huile de tournesol, l'huile de germes de blé, l'huile de germes de maïs, l'huile de soja, l'huile de coton, l'huile de luzerne, l'huile de pavot, l'huile de potiron, l'huile d'onagre, l'huile de millet, l'huile d'orge, l'huile de seigle, l'huile de carthame, l'huile de bancoulier, l'huile de passiflore, l'huile de noisette, l'huile de palme, le beurre de karité, l'huile de noyau d'abricot, l'huile de calophyllum, l'huile de sysymbrium, l'huile d'avocat, l'huile de calendula ; les huiles végétales éthoxylées ; les huiles synthétiques comme les esters d'acides gras tels que le myristate de butyle, le myristate de propyle, le myristate de cétyle, le palmitate d'isopropyle, le stéarate de butyle, le stéarate d'hexadécyle, le stéarate d'isopropyle, le stéarate d'octyle, le stéarate d'isocétyle, l'oléate de dodécyle, le laurate d'hexyle, le dicaprylate de propylèneglycol, les esters dérivés d'acide lanolique, tels que le lanolate d'isopropyle, le lanolate d'isocétyle, les monoglycérides, diglycérides et triglycérides d'acides gras comme le triheptanoate de glycérol, les alkylbenzoates, les polyalphaoléfines, les polyoléfines comme le polyisobutène, les isoalcanes de synthèse comme l'isohexadecane, l'isododécane, les huiles perfluorées et les huiles de silicone. Parmi ces dernières, on peut plus particulièrement citer les diméthylpolysiloxanes, méthylphénylpolysiloxanes, les silicones modifiées par des amines, les silicones modifiés par des acides gras, les silicones modifiés par des alcools, les silicones modifiés par des alcools et des acides gras, des silicones modifiés par des groupements polyéther, des silicones époxy modifiés, des silicones modifiées par des groupements fluorés, des silicones cycliques et des silicones modifiées par des groupements alkyles.

Comme autre matière grasse que l'on peut associer à cet actif, on peut citer les alcools gras ou les acides gras.

Parmi les polymères épaississants et/ou émulsionnant utilisés dans la présente invention, il y a par exemple, les homopolymères ou copolymères de l'acide acrylique ou de dérivés de l'acide acrylique, les homopolymères ou copolymères de l'acrylamide, les homopolymères ou copolymères de dérivés de l'acrylamide, les homopolymères ou copolymères de l'acide acrylamidométhyl propanesulfonique, de monomère vinylique, de chlorure de triméthylaminoéthylacrylate, les hydrocolloïdes d'origine végétale ou biosynthétique, par exemple la gomme de xanthane, la gomme de karaya, les carraghénates, les alginates ; les silicates ; la cellulose et ses dérivés ; l'amidon et ses dérivés hydrophiles ; les polyuréthanes. Parmi les polymères de type polyélectrolytes, pouvant être mis en jeu dans la production d'une phase aqueuse gélifiée apte à être utilisée dans la préparation d'émulsions E/H, H/E, E/H/E ou H/E/H, ou d'un gel aqueux contenant les extraits de quinoa objets de la présente invention, il y a par exemple, les copolymères de l'acide acrylique et de l'acide-2-méthyl-[(1-oxo-2-propényl)amino] 1-propane sulfonique (AMPS), les copolymères de l'acrylamide et de l'acide-2-méthyl-[(1-oxo-2-propènyl)amino] 1-propane sulfonique, les copolymères de l'acide-2-méthyl-[(1-oxo-2-propényl)amino] 1-propane sulfonique et de l'acrylate de (2-hydroxyéthyle), l'homopolymère de l'acide-2-méthyl-[(1-oxo-2-propényl)amino] 1-propane sulfonique, l'homopolymère de l'acide acrylique, les copolymères du chlorure d'acryloyl éthyl triméthyl ammonium et de l'acrylamide, les copolymères de l'AMPS et de la vinylpyrolidone, les copolymères de l'acide acrylique et d'acrylates d'alkyle dont la chaîne carbonée comprend entre dix et trente atomes de carbone, les copolymères de l'AMPS et d'acrylates d'alkyle dont la chaîne carbonée comprend entre dix et trente atomes de carbone. De tels polymères sont commercialisés respectivement sous les appellations SIMULGEL™ EG, SEPIGEL™ 305, SIMULGEL™ NS, SIMULGEL™ 800, SIMULGEL™ A, SIMULGEL™ EPG, SIMULGEL™ INS, SIMULGEL™ FL, SEPIGEL™ 501, SEPIGEL™ 502, SEPIPLUS™ 250, SEPIPLUS™ 265, SEPIPLUS™ 400, SEPINOV™ EMT 10, CARBOPOL™, ULTREZ™ 10, ACULYN™ , PEMULEN™ TR1, PEMULEN™ TR2, LUVIGEL™ EM, SALCARE™ SC91, SALCARE™ SC92, SALCARE™ SC95, SALCARE™ SC96, FLOCARE™ ET100, FLOCARE™ ET58, HISPAGEL™, NOVEMER™ EC1, ARISTOFLEX™ AVC, ARISTOFLEX™ HBM, RAPITHIX™ A60, RAPITHIX™ A100, COSMEDIA SP et STABILEZE™ 06..

Parmi les cires utilisables dans la présente invention, on peut citer par exemple la cire d'abeille ; la cire de carnauba ; la cire de candelilla ; la cire d'ouricoury ; la cire du Japon ; la cire de fibre de liège ou de canne à sucre ; les cires de paraffines ; les cires de lignite ; les cires microcristallines ; la cire de lanoline ; l'ozokérite ; la cire de polyéthylène ; les huiles hydrogénées ; les cires de silicone ; les cires végétales ; les alcools gras et les acides gras solides à température ambiante ; les glycérides solides à température ambiante.

Parmi les émulsionnants utilisables dans la présente invention, on peut citer :
- les esters gras d'alkylpolyglycosides éventuellement alkoxylés, et tout particulièrement les esters de méthylpolyglucoside éthoxylés tels que le PEG 120 méthyl glucose trioléate et le PEG 120 méthyl glucose dioléate commercialisés respectivement sous les appellations GLUCAMATE™ LT et GLUMATE™ DOE120.
- Les esters gras alkoxylés tels que le PEG 150 pentaérythrytyl tétrastéarate commercialisé sous l'appellation CROTHIX™ DS53, le PEG 55 propylene glycol oléate commercialisé sous l'appellation ANTIL™ 141.
- Les carbamates de polyalkylène glycols à chaînes grasses tels que le PPG 14 laureth isophoryl dicarbamate commercialisé sous l'appellation ELFACOS™ T211, le PPG 14 palmeth 60 hexyl dicarbamate commercialisé sous l'appellation ELFACOS™ GT2125.
- Les acides gras, les acides gras éthoxylés, les esters d'acide gras et de sorbitol, les esters d'acides gras éthoxylés, les polysorbates, les esters de polyglycérol, les alcools gras éthoxylés, les esters de sucrose, les alkylpolyglycosides, les alcools gras sulfatés et phosphatés ou les mélanges d'alkylpolyglycosides et d'alcools gras décrits dans les demandes de brevet français 2 668 080, 2 734 496, 2 756 195, 2 762 317, 2 784 680, 2 784 904, 2 791 565, 2 790 977, 2 807 435, 2 804 432, 2 830 774, 2 830 445, les associations de tensioactifs émulsionnants choisis parmi les alkylpolyglycosides, les associations d'alkylpolyglycosides et d'alcools gras, les esters de polyglycérols ou de polyglycols ou de polyols tels que les polyhydroxystéarates de polyglycols ou de polyglycérols mis en oeuvre dans les demandes de brevets français 2 852 257, 2 858 554, 2 820 316 et 2 852 258.

Comme exemples de principe actif que l'on peut associer aux extraits de quinoa, on peut citer les composés ayant une action éclaircissante ou dépigmentante tels que par exemple l'arbutine, l'acide kojique, l'hydroquinone, l'acide ellagique, la vitamine C, le magnésium ascorbyl phosphate, le SEPICALM™ MSH, les extraits de polyphénols, les extraits de raisin, les extraits de pin, les extraits de vin, les extraits d'olives, les extraits de mare, les protéines N-acylées, les peptides N-acylés, les acides aminés N-acylés, comme par exemple le N-lauroyl proline, le N-linoléyl lysine, le N-linoléyl leucine, le N-octanoyl glycine, le N-undécylénoyl phénylalanine, le N-palmitoyl proline, les hydrolysâts partiels de protéines N-acylés, les acides aminés, les peptides, les hydrolysâts totaux de protéines, les hydrolysâts partiels de protéines, les polyols (par exemple, la glycérine ou le butylène glycol) , l'urée, l'acide pyrrolidonecarboxylique ou les dérivés de cet acide, l'acide glycyrrhétinique, l'alpha-bisabolol, les sucres ou les dérivés des sucres, les polysaccharides ou leurs dérivés, les hydroxyacides par exemple l'acide lactique, les vitamines, les dérivés de vitamines comme le Rétinol, la vitamine E et ses dérivés, les minéraux, les enzymes, les co-enzymes, comme, le Coenzyme Q10, les hormones ou "hormone like", les extraits de soja par exemple, la Raffermine™, les extraits de blé par exemple la Tensine™ ou la Gliadine™, les extraits végétaux, tels que les extraits riches en tanins, les extraits riches en isoflavones ou les extraits riches en terpènes, les extraits d'algues d'eau douce ou marines, les cires essentielles, les extraits bactériens, les minéraux, les lipides en général, les lipides tels que les céramides ou les phospholipides, les actifs ayant une action amincissante comme la caféine ou ses dérivés, les actifs ayant une activité anti-microbienne ou une action purifiante vis à vis des peaux grasses tels que le LIPACIDE™ PVB, le LIPACIDE™ UG, les actifs ayant une propriété énergisante ou stimulante comme le SEPITONIC™ M3 ou le Physiogényl™ le panthénol et ses dérivés comme le SEPICAP™ MP, les actifs anti-age comme le SEPILIFT™ DPHP, le LIPACIDE™ PVB, le SEPIVINOL™, le SEPIVITAL™, les actifs hydratants comme le SEPICALM™ S, le SEPICALM™ VG et le LIPACIDE™ DPHP, le PROTEOL™ SAV 50, les actifs anti-age " anti-photo vieillissement ", les actifs protecteurs de l'intégrité de la jonction dermo-épidermique, les actifs augmentant la synthèse des composants de la matrice extracellulaire, les actifs ayant une activité amincissante, raffermissant ou drainante comme la caféine, la théophylline, l'Adénosyl Mono Phosphate cyclique (AMPc), le thé vert, la sauge, le ginko biloba, le lierre, le marron d'inde, le bambou, le ruscus, le petit houx, la centella asiatica, la bruyère, l'ulmaine, le fucus, le romarin, la saule , des actifs créant une sensation de « chauffe » sur la peau comme les activateurs de la microcirculation cutanée (exemple des nicotinates) ou des produits créant une sensation de « fraîcheur » sur la peau (exemple du menthol et des dérivés).

Comme filtre solaire que l'on peut incorporer dans la composition selon l'invention, on peut citer tous ceux figurant dans la directive cosmétique 76/768/CEE modifiée annexe VII.

L'étude expérimentale suivante illustre l'invention sans toutefois la limiter.

### A - Evaluation in vitro de l'activité lipolytique des extraits de quinoa dans des cultures d'adipocytes humains normaux par dosage des acides gras libres

### 1 - But et principe de la méthode

L'expérience à pour objet de mettre en évidence, dans un modèle in vitro d'adipocytes humains isolés, l'activité lipolytique des composés mis en oeuvre. L'hydrolyse des triglycérides en acides gras non estérifiés et en glycérol est appelée lipolyse. Les triglycérides sont stockés dans les adipocytes et constituent la réserve graisseuse. Pour que cette réserve soit diminuée, ce qui est la finalité recherchée lorsque l'on utilise des produits amincissants, les triglycérides doivent être hydrolysés sous forme d'acides gras, qui eux peuvent être éliminés de la cellule. L'hydrolyse des triglycérides met en jeu une lipase hormonodépendante qui doit être phosphorylée pour être active. L'étape de phosphorylation met en jeu une kinase et l'Adénosyl Mono Phosphate cyclique (AMPc). Une augmentation du contenu en AMPc des adipocytes est nécessaire pour favoriser l'activité de la lipase et donc la lipolyse. La méthode décrite consiste en une incubation des produits en présence d'adipocytes humains en suspension, suivie d'une mesure du taux intracellulaire en AMPc.

### 2 - Protocole expérimental

**(i)** - Modèle cellulaire : Le test est réalisé à partir d'adipocytes humains isolés et préparés en suspension cellulaire. Les adipocytes sont isolés à partir du tissu adipeux abdominal sous-cutané récupéré lors d'opérations de chirurgies esthétiques (plasties abdominales) réalisées sur des femmes. Les cellules sont isolées à partir du tissu frais. Le tissu adipeux est isolé et dissocié par action d'une collagénase (SIGMA™, 1 mg/ml, 30 minutes à 37°C, agitation douce). La collagénase digère le tissu conjonctif présent dans le tissu adipeux. Après digestion, les cellules sont filtrées et lavées dans un milieu de culture approprié contenant le milieu MEM (Minimum Essential Medium en langue anglaise) sans rouge de phénol, sans glutamine (SIGMA) + 2,2 mg/ml de bicarbonate de sodium (GIBCO) + 50 UI de pénicilline (BIOWHITTAKER™) + 50 µg/ml de streptomycine (BIOWHITTAKER™) + 1% (v/v) de L-glutamine (BIOWHITTAKER™) + 0,5% d'albumine sérique délipidée (SIGMA™). La suspension d'adipocytes est utilisée immédiatement après sa préparation.
**(ii)** - Incubation des produits avec les adipocytes : Les produits à tester sont dilués dans le milieu de culture des adipocytes. Ils sont incubés avec les cellules en suspension pendant deux heures à 37°C (250 µl de produit + 250 µl de suspension d'adipocytes). L'extrait glycolique de Quinoa, commercialisé en France par la société PROVITAL comprend entre 0,5% et 1% massique d'extrait sec; il est testé à 0,0001% massique et 0,0005% massique d'extrait sec (ES).

### 3 - Evaluation des résultats

**(i) -** Concentration en acides gras libres : A l'issue de l'incubation, la lyse cellulaire est contrôlée à vue, par la présence d'une couche lipidique à la surface de la suspension cellulaire. Les milieux sous-nageants sont prélevés. Les acides gras libres sont dosés par spectrophotométrie à l'aide d'un kit commercial, (kit NEFA™ C, WAKO), par référence à une gamme étalon d'acides gras. L'activité lipolytique des produits est évaluée par rapport à un groupe témoin incubé en présence des adipocytes et en absence de produit. La réactivité des adipocytes est systématiquement contrôlée pour la mesure de l'activité lipolytique des produits de référence, la caféine (1,3,7-triméthyl xanthine) et la théophylline (1,3-diméthyl 2,6-dihydroxy purine). On réalise cinq dosages pour chacun des produits testés.
**(ii) -** Les résultats des essais, exprimés en moyenne arithmétique des cinq dosages réalisés pour chacun des produits, sont consignés dans le tableau suivant :

| | Concentration d'incubation (% poids ES) | Concentration en acides gras libres (µmole) | activité lipolytique (par rapport au témoin = 100) |
|---|---|---|---|
| Témoin | - | 16 ± 3 | 100 |
| Caféine | 0,0001 | 25 ± 4 | 154 |
| Extrait glycolique de quinoa Théophylline | 0,0001 | 20 ± 3 | 126 |
| Extrait glycolique de quinoa | 0,0005 | 23 ± 1 | 141 |

| | | | |
|---|---|---|---|
| ES : extrait sec | | | |

Ces résultats font apparaître que l'extrait de quinoa présente une activité lipolytique de même ordre de grandeur que celle de la caféine, ce qui en fait un candidat comme principe actif de compositions amincissantes.

### B - Mesure de la différenciation des pré-adipocytes en adipocytes, recherche d'un effet inhibiteur

### 1 - Principe de la méthode

La méthode décrite consiste en une incubation du produit en présence de pré-adipocytes murins (lignée 3T3-L1) qui, sous l'influence d'un milieu de culture approprié, se différencient en adipocytes matures capables de stocker des triglycérides. La différenciation est évaluée par un dosage enzymatique de la Glucose-6-phosphate deshydrogénase (G6PDH). Cette enzyme, localisée à l'intérieur des adipocytes, permet la conversion du Glucose-6-phosphate en Gluconate-6-phosphate en présence de Nicotinamide Adenine Dinucléotide Phosphate (NADP) et conduit à la formation de Nicotinamide Adenine Dinucléotide Phosphate Hydrogéné (NAPDH) utilisé pour la néosynthèse d'acides gras. Elle est présente dans les adipocytes matures. La formation de NAPDH est suivie par spectrophotométrie d'absorption, à 340 nanomètres.

### 2 - Protocole expérimental

**(i)** - Modèle cellulaire : Le test est réalisé à partir de pré-adipocytes murins (lignée cellulaire 3T3-L1) cultivés en mono-couche jusqu'à confluence. Pour cela, les cellules (60 10³ cellules / cm²) sont ensemencées et cultivées pendant 5 jours dans du milieu DMEM à 4,5 g/l de glucose (SIGMA) + 10% sérum foetal (SIGMA) + 50 UI de pénicilline (BIOWHITTAKER™) + 50 µg/ml de streptomycine (BIOWHITTAKER™) + 1% (v/v) de L-glutamine (BIOWHITTAKER™) (milieu A). Deux jours après la confluence de cellules, celles-ci sont incubées dans un milieu de différentiation composé du milieu A additionné de 0,1 µM de dexaméthasone + 0,17 M d'insuline + 0,5 mM d'isobutylméthylxanthine (milieu B). Les cellules sont incubées dans ce milieu pendant deux jours puis deux jours supplémentaires dans le milieu A additionné de 0,17 M d'insuline (milieu C). Les cellules sont enfin cultivées 5 jours supplémentaires dans le milieu A. en suspension pendant deux heures à 37°C (250 µl de produit + 250 µl de suspension d'adipocytes). L'extrait glycolique de Quinoa, commercialisé en France par la société PROVITAL, est testé à 0,0001% massique et 0,0005% massique d'extrait sec (ES).
**(ii)** - Incubation du produit avec les pré-adipocytes : L'extrait glycolique de Quinoa à 0,02 % ES est incubé au moment de l'initiation de la différenciation par le milieu B. Il est ensuite incubé jusqu'au moment du dosage.
**(iii) -** Dosage de l'activité de la G6PDH : A l'issue de l'incubation, les cellules sont lavées dans un tampon Tris-EDTA-HCL (50 niniole.l⁻¹ / 5mmole.l⁻¹, pH = 7,5) puis lysées par action d'ultrasons. Le lysat cellulaire ainsi obtenu est conservé au frais. L'activité de la G6PDH des lysats cellulaires est mesurée par ajout de son substrat, le D-glucose 6P (1 mmole) et de son cofacteur le NADP (8 mmoles). La cinétique d'apparition du NAPDH est mesurée par spectrophotométrie à 340 nm pendant deux minutes. Le dosage se fait contre une gamme étalon de G6PDH. L'effet du produit sur la différenciation des pré-adipocytes est évalué par rapport à un groupe témoin incubé en présence des pré-adipocytes et en absence de produit (témoin différencié) ainsi que par rapport à un groupe témoin non différencié, incubé en présence des pré-adipocytes cultivés uniquement dans le milieu A.

| | Concentration d'incubation (% poids ES) | G6PDH (U/ml) | % d'inhibition |
|---|---|---|---|
| Témoin non différencié | - | 0,03 ± 0,002 | 100% |
| Témoin différencié | 0,0000001 | 0,46 ± 0,03 | 0% |
| TNFα (Tumorial Necrosis Factor) | 0,0000001 | 0,005 ± 0,01 | 43 |
| Extrait glycolique de quinoa | 0,02 | 0,35 ± 0,03 | 26 |

| | | | |
|---|---|---|---|
| ES : extrait sec | | | |

Le TNFα testé à 1 ng/ml (soit 10⁻⁷% ES), inhibe de 43% la différenciation des pré-adipocytes. L'extrait glycolique de quinoa testé, inhibe de 26% l'activité de la G6PDH ce qui le rend approprié à une utilisation comme principe actif amincissant en prévenant la formation de graisse dans le corps humain..

### C) - Exemples de formulations cosmétiques

Dans les exemples suivants les proportions sont exprimées en pourcentages pondéraux.

### Exemple 1 : Lait corporel amincissant

| | |
|---|---|
| MONTANOV™ L | 3,00 % |
| Phytosqualane | 8,00 % |
| Huile d'amandes douces | 2,00 % |
| Eau | qsp 100 % |
| SEPIGEL™ 501 | 1,50 % |
| Extrait glycolique de Quinoa | 3,00 % |
| SEPICIDE™ CI | 0,20 % |
| SEPICIDE™ HB | 0,30 % |
| Parfum | 0,30 % |

### Exemple 2 : Crème anti-relâchement (cible ovale du visage)

| | |
|---|---|
| MONTANOV™ 202 | 3,50 % |
| MONTANOV™ 14 | 1,00 % |
| SEPILIFT™ DPHP | 1,00 % |
| LANOL™ 1688 | 15,00 % |
| Huile de germes de blé | 5, 00 % |
| Eau | qsp 100% |
| SIMULGEL™ EG | 1,30 % |
| Extrait glycolique de Quinoa | 2,00 % |
| SEPICIDE™ CI | 0,20 % |
| SEPICIDE™ HB | 0,30 % |
| Parfum | 0,10 % |

### Exemple 3 : Atomiseur anti-rondeurs

| | |
|---|---|
| MONTANE™ 60 | 3,30 % |
| MONTANOX™ 60 | 1,70 % |
| Caprilic / capric triglycérides | 6,00 % |
| Isohexadecane | 5,00 % |
| Magnésium Aluminium Silicate | 1,50% |
| Eau | qsp 100 % |
| SIMULGEL™ EG | 1,00 % |
| Extrait glycolique de Quinoa | 2,00 % |
| Centella asiatica / extrait d'hydrocotyles | 1,00 % |
| SEPICIDE™ CI | 0,20 % |
| SEPICIDE™ HB | 0,30 % |
| Parfum | 0,40 % |
| Eau | qsp 100 % |

### Exemple 4 : Gel amincissant fraîcheur

| | |
|---|---|
| SEPIGEL™ 305 | 3,50 % |
| Hydroxyéthyl cellulose | 1,00 % |
| Caféine | 5,00 % |
| Menthol | 0,30 % |
| Ethanol | 50,00 % |
| Extrait glycolique de Quinoa | 3,00 % |
| SEPICIDE™ LD | 1,00 % |
| Parfum | 0,20 % |
| Eau | qsp 100 % |

### Exemple 5 : Fluide corporel minceur

| | |
|---|---|
| SIMULGEL™ NS | 2,50 % |
| Gomme de xanthane | 0,20 % |
| LANOL™ 99 | 5,00 % |
| Extrait glycolique de Quinoa | 2,00 % |
| Extrait de Ginkgo Biloba | 2,00 % |
| Extrait de cola | 1,00 % |
| Extrait de Ginseng | 0,50 % |
| SEPICIDE™ HB | 1,50 % |
| Parfum | 0,10 % |
| Eau | qsp 100 % |

### Exemple 6 : Lotion revitalisante fermeté destinée à être imprégnée sur des lingettes corporelles

| | |
|---|---|
| Extrait glycolique de Quinoa | 1,50 % |
| Glycérine | 5,00 % |
| Ethanol | 5,00 % |
| Extrait de Ruscus | 3,00 % |
| SEPITONIC™ M3 | 1,00 % |
| SEPICIDE™ CI | 0,20 % |
| SEPICIDE™ HB | 0,30 % |
| Eau | qsp 100 % |

### Exemple 7 : gel douche amincissant

| | |
|---|---|
| MONTALINE™ C40 | 8,00 % |
| PROTEOL™ OAT | 5,00 % |
| Sodium lauryl sulfate | 9,00 % |
| Extrait glycolique de Quinoa | 3,00 % |
| Extrait de thé vert | 1,00 % |
| KATHON™ CG | 0,80 % |
| Colorant vert | qs |
| Parfum de thé vert | 1,00 % |
| Acide lactique | qs PH=6,5 |
| Eau | qsp 100% |

### Exemple 8 : Massage désinfiltrant biphasique

| | |
|---|---|
| Huile de café arabica | 1,00 % |
| LANOL™ 189 | 20,00 % |
| LANOL™ 99 | 10,00 % |
| Huile de bourrache | 2,00 % |
| Parfum | 0,10 % |
| Extrait glycolique de Quinoa | 3,00 % |
| Glycérine | 3,00 % |
| Ethanol | 10,00 % |
| Colorant bleu | qs |
| Eau | qsp 100 % |

Les définitions des produits commerciaux utilisés dans les exemples sont les suivantes :
SEPILIFT™ DPHP : (Dénomination INCI : Dipalmitoyl hydroxyproline), commercialisé par la société SEPPIC ;
SEPICIDE™ CI : Imidazoline urée, (agent conservateur) commercialisé par la société SEPPIC ;
SEPICIDE™ HB : Mélange de phénoxyéthanol, de méthylparaben, d'éthylparaben, de propyl-paraben et de butylparaben, (agent conservateur) commercialisé par la société SEPPIC ;
SEPICIDE™ LD : phénoxyéthanol commercialisé par la société SEPPIC ;
KATHON™ CG : (Dénomination INCI: méthyl isothiazolinone / Méthyl chloroisothiazolinone) ;
MONTANE™ 60 : Sorbitan stéarate ;
MONTANOX™ 60 : Polysorbate 60 ;
SIMULGEL™ EG : Latex inverse auto-inversible de copolymère tel que ceux décrits dans la publication internationale WO 99/36445 (dénomination INCI : Sodium acrylate/Sodium acryloyldimethyl taurate copolymer et Isohexadecane et Polysorbate 80) commercialisé par la société SEPPIC ;
SIMULGEL™ NS : Latex inverse auto-inversible de copolymère tel que ceux décrits dans la publication internationale WO 99/36445 (dénomination INCI : hydroxyethylacrylate/Sodium acryloyldimethyl taurate copolymer et squalane et Polysorbate 60 ) commercialisé par la société SEPPIC ;
SEPIGEL™ 305 : Latex inverse auto-inversible (dénomination INCI : Polacrylamide /C13-14 Isoparaffin / Laureth-7) ;
SEPIGEL™ 501 : Latex inverse auto-inversible dénomination INCI : C13-14 Isoparaflin/Mineral Oil/Sodium polyacrylate/Polyacrylamide/Polysorbate 85) commercialisé par la société SEPPIC ;
LANOL™ 99 : Isononanoate d'isononyle commercialisé par la société SEPPIC ;
LANOL™ 189: Isostearyl isononanoate ;
LANOL™ 1688 : Ethylhexanoate de cétéaryle commercialisé par la société SEPPIC ;
SEPITONIC™ M3 : Mélange d'aspartate de magnésium, de gluconate de cuivre et de gluconate de zinc commercialisé par la société SEPPIC ;
MONTALINE™ C40 : Cocamidopropyl betainamide MEA chloride ;
PROTEOL™ OAT : Acides aminés d'avoine N'lauroylé oat amino acids ;
MONTANOV™ 14 : Myristyl alcohol / Myristyl glucoside ;
MONTANOV™ L : Agent émulsionnant à base d'alcool en C14-C22 et d'alkyl polyglucoside en C12-C20 tel que ceux décrits dans la demande de brevet européen EP 0 995 487 ;
MONTANOV™ 202 est un agent émulsionnant à base d'alcool arachidylique d'alcool béhènylique et d'arachidyl polyglucoside.

## Revendications

1. Utilisation d'au moins un extrait de Quinoa, comme actif amincissant et/ou comme actif prévenant de la formation de nouvelles graisses dans le corps humain, dans une composition contenant un milieu cosmétiquement acceptable.

2. Procédé de traitement non thérapeutique du corps humain destiné à l'amincir, **caractérisé en ce que** l'on y applique une composition contenant un milieu cosmétiquement acceptable et une quantité efficace d'au moins un extrait de Quinoa.

3. Utilisation d'au moins un extrait de Quinoa, pour préparer un médicament à activité lipolytique, destiné à induire l'amincissement du corps humain.

4. Utilisation d'au moins un extrait de Quinoa, pour préparer un médicament à activité inhibitrice de différenciation des pré-adipocytes, destiné à prévenir la formation de nouvelles graisses du corps humain et/ou animal.

## Claims

1. Use of at least one quinoa extract, as a slimming active agent and/or as an active agent which prevents the formation of new fat in the human body, in a composition containing a cosmetically acceptable medium.

2. Method for the nontherapeutic treatment of the human body intended for slimming it, **characterized in that** a composition containing a cosmetically acceptable medium and an effective quantity of at least one quinoa extract is applied thereto.

3. Use of at least one quinoa extract, for preparing a medicament with lipolytic activity, intended to induce slimming of the human body.

4. Use of at least one quinoa extract, for preparing a medicament with inhibitory activity on the differentiation of the preadipocytes, intended to prevent the formation of new fat in the human and/or animal body.

## Patentansprüche

1. Verwendung von mindestens einem Quinoa-Extrakt als Schlankheitsmittelwirkstoff und/oder als Wirkstoff zur Verhinderung der Fettneubildung im menschlichen Körper in einer Zusammensetzung, die ein kosmetisch unbedenkliches Medium enthält.

2. Verfahren für die nichttherapeutische Behandlung des menschlichen Körpers für die Gewichtsabnahme, **dadurch gekennzeichnet, dass** man eine Zusammensetzung, die ein kosmetisch unbedenkliches Medium und eine wirksame Menge von mindestens einem Quinoa-Extrakt enthält, auf diesen appliziert.

3. Verwendung von mindestens einem Quinoa-Extrakt zur Herstellung eines Arzneimittels mit lipolytischer Wirksamkeit zur Induktion von Gewichtsabnahme beim menschlichen Körper.

4. Verwendung von mindestens einem Quinoa-Extrakt zur Herstellung eines Arzneimittels mit Hemmwirkung auf die Differenzierung der Präadipozyten zur Verhinderung der Fettneubildung beim menschlichen und/oder tierischen Körper.
